# EUROPEAN PATENT SPECIFICATION

(11) **EP 0 667 160 B1**
(45) Date of publication and mention of the grant of the patent: **02.05.2002**
(21) Application number: 94119571.1
(22) Date of filing: 10.12.1994
(51) Int. Cl.: A61K 31/557, C07C 405/00

(54) **Use of certain prostaglandin analogues to treat glaucoma and ocular hypertension**
Anwendung von Prostagrandin-Analogen für die Behandlung von Glaukome und okuläre Hypertension
Utilisation d'analogues de prostaglandines pour le traitement de hypertension oculaire et le glaucome

(30) Priority: 15.12.1993 US 167470; 15.12.1993 US 167747; 30.09.1994 US 316672
(43) Date of publication of application: 16.08.1995
(60) Divisional application: 00204408.9
(73) Proprietor: ALCON LABORATORIES, INC., Fort Worth Texas 76134-2099 (US)
(72) Inventor: Sallee, Verney L., Burleson, Texas 76028 (US); DeSantis, Louis, Jr., Fort Worth, Texas 76109 (US); Zinke, Paul W., Fort Worth, Texas 76133 (US); Bishop, John E., Groton, Massachusetts 01450 (US); Klimko, Peter G., Fort Worth, Texas 76110 (US); Selliah, Robert D., Fort Worth, Texas 76132 (US); Dean, Thomas R., Weatherford, Texas 76087 (US); Hellberg, Mark R., Arlington, Texas 76017 (US)
(74) Representative: Hall, Marina

(56) References cited:
- EP-A- 0 299 914
- WO-A-86/04504
- WO-A-86/05488
- WO-A-92/02495
- WO-A-92/08697
- WO-A-94/05631
- DE-A- 4 229 053
- TETRAHEDRON LETTERS, OXFORD GB,
- PROSTAGLANDINS, vol. 35, no. 6, STONEHAM, MA US, pages 855-868, K.H. THIERAUCH ET AL. 'STABLE 9A- OR 11B-HALOGEN -15- CYCLOHEXYL-PG'S WITH HIGH AFFINITY TO THE PGD2-RECEPTOR.'
- Woodward, D. F. et al., Investigative Ophtal. and Visual Sci., 31(1), 1990, 138-46
- Graephe's Arch. Clin. Exp. Ophtalmol., 229, 1991, 411-13

## Description

### Background of the Invention

The present invention relates to the use of certain prostaglandins and prostaglandin analogues for the treatment of glaucoma and ocular hypertension. As used herein, the terms "prostaglandin" and "PG" shall refer to prostaglandins and derivatives and analogues thereof, except as otherwise indicated by context.

Naturally-occurring prostaglandins are known to lower intraocular pressure (IOP) after topical ocular instillation, but generally cause inflammation, as well as surface irritation characterized by conjunctival hyperemia and edema. Many synthetic prostaglandins have been observed to lower intraocular pressure, but such compounds also produce the aforementioned side effects. Various attempts have been made to overcome the ocular side effects associated with prostaglandins. Stjernschantz et al. (EP 364 417 B1) have synthesized derivatives or analogues of naturally-occurring prostaglandins in order to design out selectively the undesired side effects while maintaining the IOP-lowering effect. Others, including Ueno et al. (EP 330 511 A2) and Wheeler (EP 435 682 A2) have tried complexing prostaglandins with various cyclodextrins.

EP-B-0 656 889 has a priority date of 31st August 1992 and was published on 17th March 1994 and is relevant only under Article 54(3) and (4) EPC. EP-B-0 656 889 discloses pharmaceutical preparations comprising esters or amides of 9-chloroprostaglandins of formula (I) 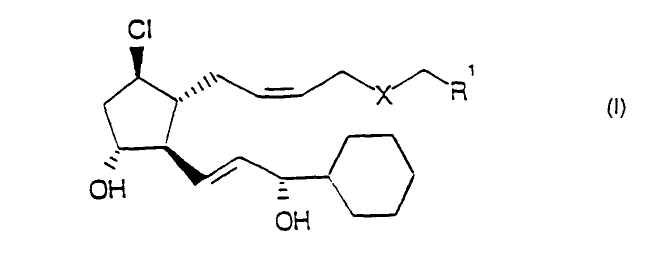 in which
- X: represents oxygen or CH₂, and
- R¹: represents COOR² wherein R² is an optionally substituted C₁-C₁₀alkyl, C₃-C₁₀-cycloalkyl, C₆-C₁₀aryl or C₆-C₁₀ar(C₁-C₄)alkyl radical. or CONHR³ wherein R³ is an optionally substituted C₁-C₁₀alkyl radical,
for treating raised intraocular pressure, and to pharmaceutical preparations comprising 9-chloroprostaglandin esters or amides of the general formula (I).

WO92/08697 relates to 9-halogen-11β-hydroxy-prostane derivatives of formula I 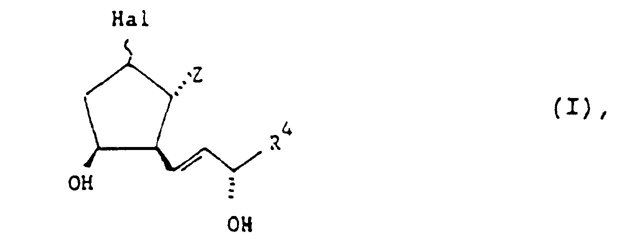 in which Z means the radicals 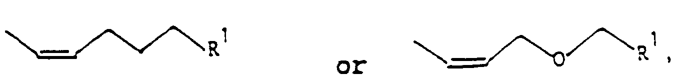 Hal means an α- or β-position chlorine or fluorine atom.
R¹ means the radical CH₂OH or 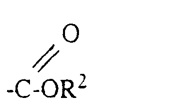 with R² meaning a hydrogen atom, an alkyl, cycloalky, aryl or heterocyclic radical or R¹ means the radical 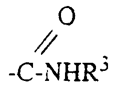 with R³ meaning an acid radical or radical R² and R⁴ means a cycloalkyl group, and if R² has the meaning of a hydrogen atom. its salts with physiologically compatible bases and its cyclodextrin clathrates,
which are especially suitable for oral administration.

WO86/04504 relates to a pharmaceutical preparation which contain as active compound cyclodextrine clathrate of prostaglandin having the general formula (I) 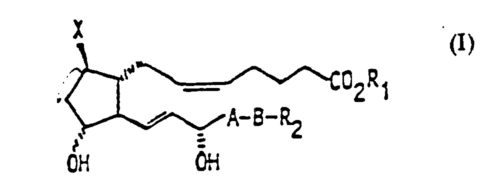 wherein R₁ is a hydrogen atom or a straight or branched chain alkyl radical having up to 10 atoms of carbon, R₂ is an alkyl, cycloalkyl, or optionally substituted phenyl group, A and B form together a direct bond or A is a straight or branched chain alkyl group having up to 10 atoms of carbon and B is an oxygen atom, a direct bond or -C = C bond and X is a chlorine or fluorine atom. The clathrate compounds according to WO86/04504 exhibit strong inhibiting activity on gastric acid secretion and protect the mucosa of the stomach against various deleterious influences, as well as against anti-inflammatory substances. Several of the clathrates according to this reference are excellently suited for the treatment of allergic and vasomotor rhinitis, and several of the clathrates are furthermore suitable, upon enteral or parenteral administration, for inducing menstruation or interrupting pregnancy. They are also suitable for synchronizing the sexual cycle of female mammals such as rabbits, cattle, horses and pigs and for cervix dilation as a preparation for diagnostic or therapeutic interventions. The clathrates of WO86/04504 can be utilized in liquid or solid galenic formulations, and the formulations can be administered enterally, parenterally, vaginally or rectally.

DE-A-4 229 053 provides esters and amides of 9-chloro-prostaglandins which have a similar spectrum of activity to the corresponding natural prostaglandins, to promote blood circulation in the skin and can be used to heal skin lesions.

EP-A-0 299 914 relates to a 9-halogen-(Z) prostaglandin derivative of formula I 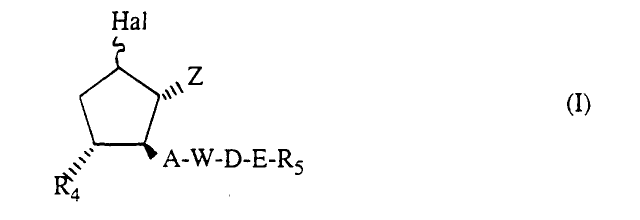 in which
Z represents the radicals 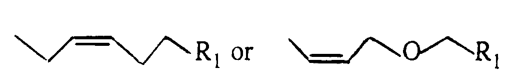 Hal represents a chlorine or fluorine atom in the alpha or beta position,
R₁ represents the radical CH₂OH or 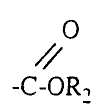 with R₂ meaning a hydrogen atom, an alkyl, cycloalkyl, aryl or heterocyclic radical or R₁ represents the radical 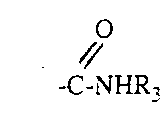 with R₃ meaning an acid radical or the radical R₂ and
A represents a -CH₂-CH₂-, a trans-CH=CH or -C≡C group,
W represents a free or a functionally modified hydroxymethylene group or a free or functionally modified 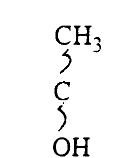 group, and the respective OH groups can be in the alpha or beta position,
D and E together represent a direct bond or
D represents a straight-chain alkylene group with 1-10 C atoms, a branchedchain alkylene group with 2-10 C atoms or an annular alkylene group with 3-10 C atoms, which optionally can be substituted by fluorine atoms, and
E represents an oxygen or sulfur atom, a direct bond. a-C≡C bond or a -CR₆=CR₇ group, and R₆ and R₇ are different and mean a hydrogen atom, a chlorine atom or a C₁-C₄ alkyl group,
R₄ represents a free or functionally modified hydroxy group,
R₅ means a hydrogen atom, an alkyl, a halogen-substituted alkyl, a cycloalkyl, an optionally substituted aryl or a heterocyclic group, and if R₂ means a hydrogen atom, its salts with physiologically compatible bases or its cyclodextrin clathrates

The 9-halogen-(Z) prostaglandins of EP-A-0 299 914 are especially suitable for oral application.

WO86/05488 relates to 9-halo-15-cycloalkyl prostaglandin derivatives of Formula I 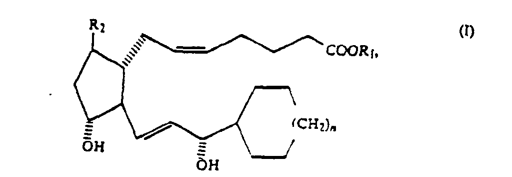 wherein
R₁ is hydrogen or methyl.
R₂ is fluorine or chlorine.
n is 0 or 1,
and, if R₁ is hydrogen, the salts thereof with physiologically compatible bases or the cyclodextrin clathrates thereof.

The compounds of WO86/05488 inhibit gastric acid secretion, exhibit a cytoprotective and ulcer-healing activity, exert a regulatory effect in cardiac arrhythmias and inhibit platelet aggregation.

WO92/02495 discloses prostaglandins derived from cyclopentanes having a fluorine atom or a hydroxy group in the 9-position of the prostane. The compounds are suitable for the treatment of diseases of the cardiovascular system, the stomach. the pancreas, the liver and the kidneys. They have a blood pressure-reducing and bronchodilatory action and are suitable for the inhibition of thrombocyte activation.

### Summary of the Invention

It has now been unexpectedly discovered that certain D series prostaglandin analogues are significantly more effective in lowering IOP than other known prostaglandins. In particular, the prostaglandin analogues of the present invention have unexpectedly been found to lower IOP to a greater degree than other known PGs without significant inflammation or associated side effects which often accompany topical ocular administration of D series prostaglandins.

### Detailed Description of the Invention

The prostaglandins which are useful in the compositions of the present invention have the general formulae (I): 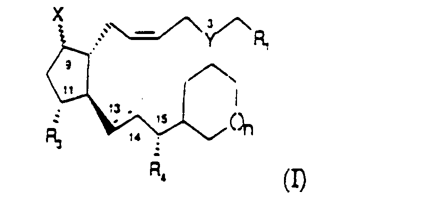 wherein:
R₁ = CO₂R₂, wherein R₂ = H, a cationic salt moiety, or an ophthalmically acceptable ammonium moiety; or R₁ may also represent an ophthalmically acceptable ester moiety;
X = halogen, particularly Cl or F, in either configuration;
Y = CH₂ or O;
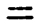 = single or trans double bond:
R₃, R₄ can be the same or different, and are selected from: free or functionally modified hydroxy groups; and
n = 0 or 1.
() represents the fact that the ring may be 5-membered or 6-membered, with the proviso that formula I is not a compound of formula 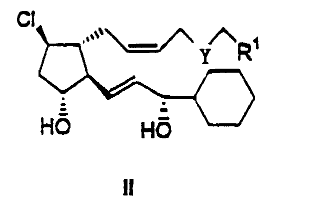 in which
Y = CH₂ or O; and
R₁ = COOR₂ in which R₂ = optionally substituted C₁₋₁₀ alkyl, C₃₋₁₀ cycloalkyl, C₆₋₁₀ aryl, or arylalkyl wherein the aryl portion contains 6-10 carbon atoms and the alkyl portion contains 1-4 carbon atoms. In addition in respect of IE, PT, SL, LT, the invention provides a topical ophthalmic composition for the treatment of glaucoma and ocular hypertension adapted only for topical ophthalmic use, to the exclusion of other topical uses or parental uses or oral uses, said composition comprising an ophthalmically acceptable vehicle and a therapeutically effective amount of a compound of formula: 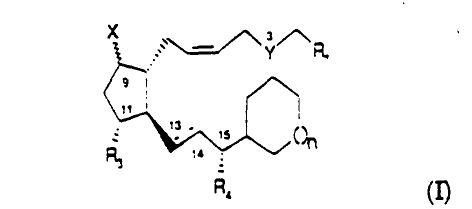 wherein
R₁ = CO₂R₂, wherein R₂ = H, a cationic salt moiety, or an ophthalmically acceptable ammonium moiety; or R₁ may also represent an ophthalmically acceptable ester moiety;
X = halogen, particularly Cl or F, in either configuration;
Y = CH₂ or O;
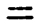 = single or *trans* double bond;
R₃, R₄ can be the same or different, and are selected from: free or functionally modified hydroxy groups;
n = 0 or 1; and
() represents the fact that the ring may be 5-membered or 6-membered.

As used in this specification, the term "ophthalmically acceptable ester moiety" refers to an ester moiety which is non-toxic and does not cause undue irritation to the ocular tissues. For compounds of formula (I), examples of ophthalmically acceptable esters include, but are not limited to: R₂ = substituted or unsubstituted alkyl, cycloalkyl, (cycloalkyl)alkyl, aryl, arylalkyl, heteroaryl, or (heteroaryl)alkyl, wherein substituents include alkyl, halo, a free or functionally modified hydroxy group, or a free or functionally modified thioL As used in this specification, the term 'heteroaryl" refers to a monocyclic ring system of 5 or 6 atoms composed of C. N, O, and/or S, such as furan, thiophene, pyrrole, pyrazole, imidazole, triazole, tetrazole, oxazole, isothiazole, thiazole, thiadiazole, pyridine, pyrimidine, pyradazine and pyrazine. The term "functionally modified hydroxy groups" refers to either etherified hydroxy groups or acylated (esterified) hydroxy groups. Similarly, the term "functionally modified thiol" refers to an alkylated thiol.

Preferred compounds of formula (I) include those wherein: R₁ = CO₂R₂; R₂ = H, methyl, ethyl, *n*-propyl, isopropyl, *t*-butyl, or benzyl; X = Cl in the β (R) configuration; Y = O or CH₂; R₃ and R₄ = OH; and n = 1. The most preferred ccmpounds of formula (I) are those wherein: R₁ = CO₂R₂; R₂ = H, methyl, ethyl, isopropyl, or t-butyl; X = Cl in the β (R) configuration; Y = O; R₃ and R₄ = OH; and n = 1.

The following Table 1 contains examples of some preferred compounds of the present invention.

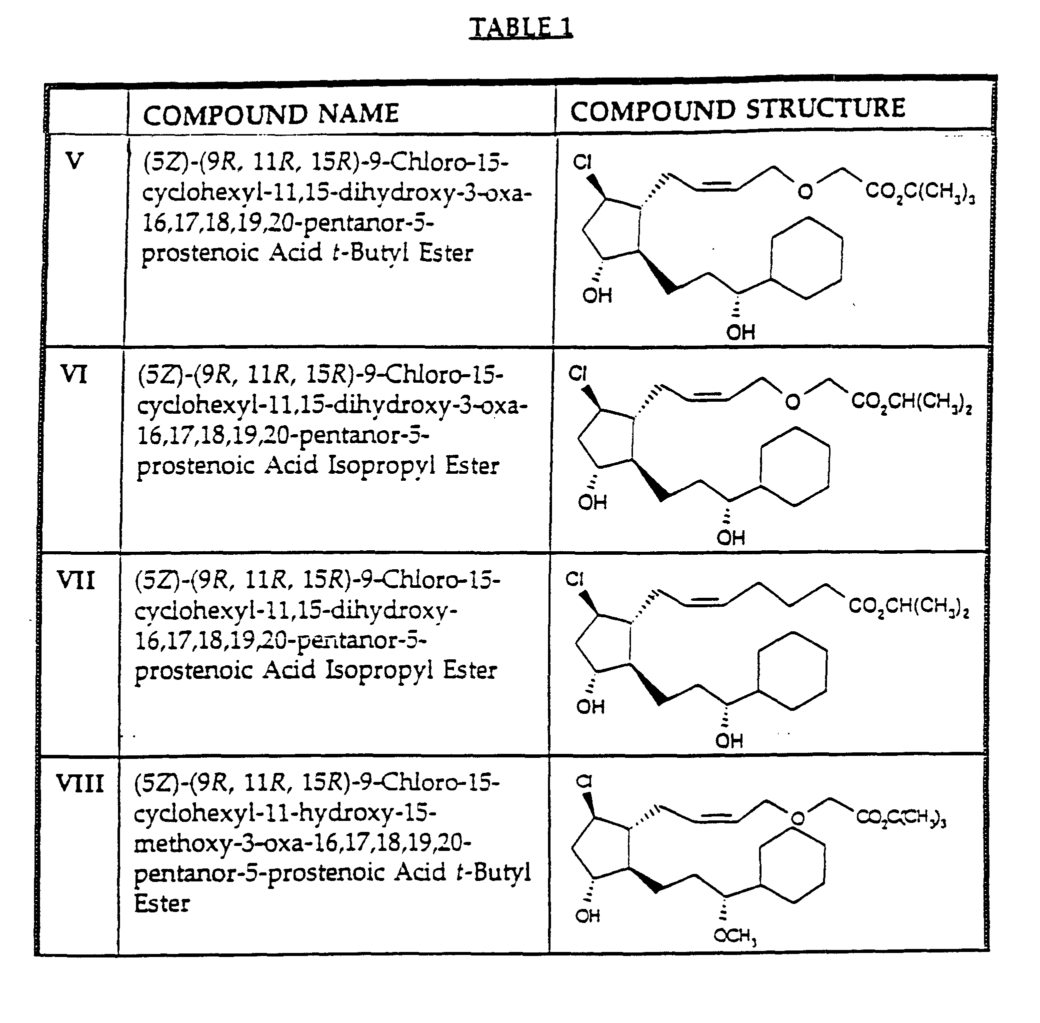

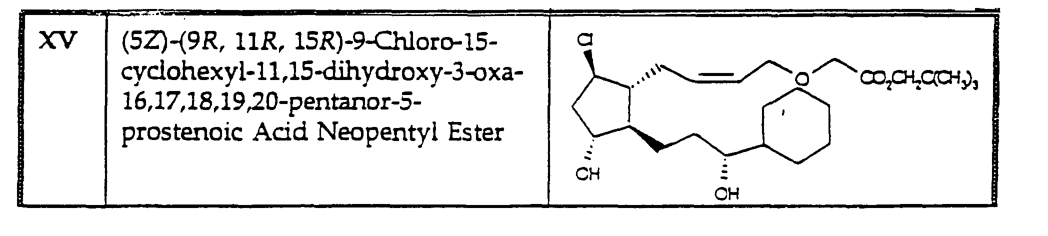

Some of the above-mentioned prostagiandins are disclosed in US 5,004,752 (Raduechel et al.) and EP 299 914 (Buchmann et al.). In addition, the syntheses of some of the prostaglandins of Table 1, above, are detailed below in Examples 1-8

In the following Examples 1-8, the following standard abbreviations are used: g = grams (mg = milligrams); mol = moles (mmol = millimoles); mol% = mole percent; ml = milliliters; mm Hg = millimeters of mercury; mp = melting point; bp = boiling point; h = hours; and min = minutes. In addition, "NMR" refers to nuclear magnetic resonance spectroscopy and "CI MS" refers to chemical ionization mass spectrometry.

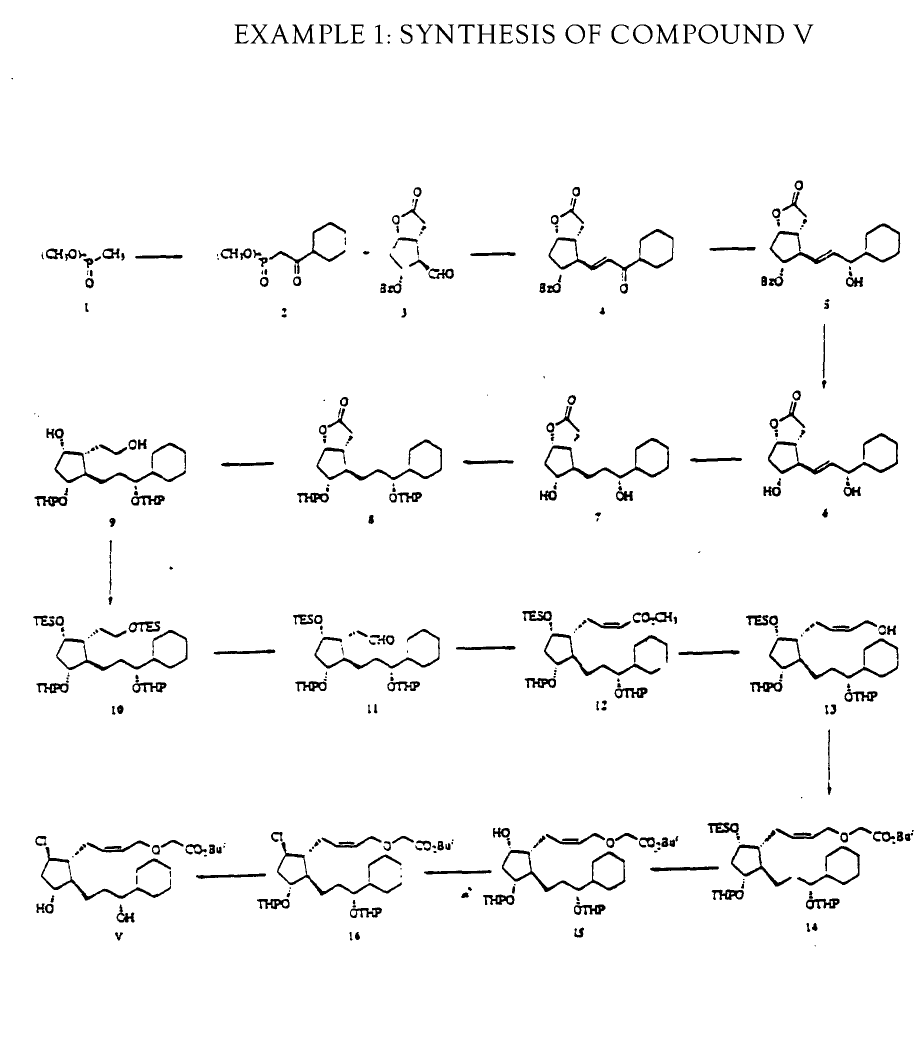

### A: Dimethyl (2-cyclohexyl-2-oxo)ethylphosphonate (2):

A solution of dimethyl methylphosphonate (100 g, 0.8 mol) in 1.0 L of anhydrous THF was cooled to -70°C and n-BuLi (2.5 M in hexanes, 320 ml, 0.8 mol) was added dropwise such that the temperature remained below -60°C. The mixture was stirred for 10 min at -70°C and then methyl cyclohexanecarboxylate (57.3 ml, 0.4 mol) was added dropwise, via syringe, over a period of 15 min. The resulting mixture was then stirred for 14 h at room temperature. The reaction was quenched by first cooling to 0°C followed by the addition of 2 M HCl until the aqueous layer was at pH 2. The layers were separated and the aqueous layer was extracted with 2 X 200 m l of CH₂Cl₂. The organic layers were combined and washed sequentially with 200 m l each of water and brine and then dried (MgSO₄). Filtration and solvent removal gave a yellow oil which was distilled under vacuum to afford 67.3 g (72%) of **2** as a clear colorless liquid: bp=100-115°C (0.01 mmHg); ¹H NMR (CDCl₃) δ 3.74 (d, *J* = 12.0 Hz, 6H), 3.08 (d, *J* = 22 Hz, 2H), 2.55 (m, 1H), 1.95 - 1.60 (m, 5H), 1.40 - 1.15 (m, 5H).

### B: (3aR, 4R, 5R, 6aS)-5-(Benzoyloxy)-4-[(E)-3-cyclohexyl-3-oxo-1-propenyl]-hexahydro-2H-cyclopenta[b]furan-2-one (4):

A solution of anhydrous THF (1.4 L), LiCl (11.7 g, 0.28 mol) and the phosphonate 2 (67.0 g, 0.28 mol) was cooled to 0°C and triethylamine (39.2 ml, 0.28 mol) was added dropwise. A solution of the aldehyde 3 (68.5 g, 0.25 mol) in dry CH₂Cl₂ (320 ml) was added dropwise to the cold suspension and the resulting mixture was stirred at 0°C for 3 h. The reaction mixture was then poured into 500 ml of 2 M HCl, and layers were separated. The aqueous layer was extracted with 500 ml of CH₂Cl₂. The combined organic layers were washed with 100 ml each of water and brine followed by drying over MgSO₄. Filtration and solvent removal gave a yellow solid which was recrystallized from EtOAc to afford 85.8 g (89%) of 4 as a white solid: mp 151-153°C; ¹H NMR (CDCl₃) δ 8.01 (d, *J =* 2.0 Hz, 2H), 7.65 - 7.40 (m, 3H), 6.70 (dd, *J* = 12, 6 Hz, 1H), 6.35,(d, *J* = 12 Hz, 1H), 5.32 (m, 1H), 5.15 (m, 1H), 2.93 (m, 3H), 2.72 - 2.25 (m, 4H), 1.85 - 1.56 (m, 6H), 1.40 - 1.15 (m, 5H).

### C: (3aR, 4R, 5R, 6aS)-5-(Benzoyloxy)-4-[(E)-(3S)-3-cyclohexyl-3-hydroxy-1-propenyl]-hexahydro-2H-cyclopenta[b]furan-2-one (5):

A solution of CeCl₃•7H₂O (19.5 g, 52.3 mmol) and enone **4** (20.0 g, 52.3 mmol) in 150 ml of CH₃OH and 70 ml of CH₂Cl₂ was prepared. NaBH₄ (1.92 g, 52.3 mmol) was added in small portions over a period of 5 min. The resulting mixture was stirred at ambient temperature for 45 min and then was poured into a separatory funnel containing 100 ml each of 25% (v/v) aqueous acetic acid and CH₂Cl₂. The layers were separated and the aqueous layer was extracted with 3 X 50 ml of CH₂Cl₂. The combined organic layers were washed with sat. NaHCO₃ (50 ml), and brine (50 ml), and then dried (MgSO₄). Upon solvent removal, 23.7 g of a colorless oil containing nearly equal amounts of the two diastereomeric allyl alcohols was obtained. The diastereomers were separated by HPLC (40% EtOAc/hexane), affording **5** (9.34 g (46%), the less polar component) as a white solid. ¹H NMR (CDCl₃) δ 8.01 (d, *J* = 8 Hz, 2H), 7.62 - 7.28 (m, 3H), 5.61 (m, 2H), 5.25 (m, 1H), 5.08 (m, 1H), 3.85 (m, 1H), 2.95 - 2.45 (m, 5H), 2.30 (m, 2H), 1.95 - 1.55 (m, 6H), 1.50 - 0.80 (m, 5H).

### D: (3aR, 4R, 5R, 6aS)-4-[(3R)-3-Cyclohexyl-3-hydroxypropyl]-hexahydro-5-hydroxy-2H-cyclopenta[b]furan-2-one (7):

A solution of the allyl alcohol **5** (10.0 g, 26.0 mmol) in warm methanol (100 ml) was cooled to ambient temperature. Anhydrous K₂CO₃ (3.6 g, 26.0 mmol) was added and the resulting mixture was stirred at ambient temperature for 3 h. The reaction mixture was concentrated and the residue was partitioned between 100 ml each of EtOAc and 1 *M* HCl. The layers were separated and the aqueous phase was extracted with 3 X 50 ml of EtOAc. The combined organic layers were washed with 50 ml of water, 2 X 50 ml of sat. NaHCO₃, 50 ml of brine, and dried over MgSO₄. Filtration and evaporation gave the diol 6 (9.8 g, 92% yield, R_{*f*} =0.26, 100% EtOAc), which was used in the subsequent reaction without further purification.

The crude diol **6** (9.8 g, 26 mmol) was dissolved in 50 ml of EtOAc and a catalytic amount (0.1 g) of 5% Pd/C was added. This mixture was hydrogenated at 30-40 psi in a Parr hydrogenation apparatus for 3 h and then filtered through a short pad of Celite. The filtrate was concentrated and the crude yellow oil was purified by passage through a short column of silica (R_{*f*} =0.26, EtOAc) to afford 7 (5.06 g, 70% yield from **5**) as a colorless, viscous oil which solidified upon standing. ¹H NMR (CDCl₃) δ 4.95 (m, 1H), 4.05 (m, 1H), 3.35 (m, 1H), 2.80 (m, 1H), 2.58 (m, 2H), 2.30 (m, 1H), 2.00 (m, 14H).

### E: (3aR, 4R, 5R, 6aS)-4-[(3R)-3-Cyclohexyl-3-(tetrahydropyran-2-yloxy)propyl]-hexahydro-5-(tetrahydropyran-2-yloxy)-2H-cyclopenta[b]furan-2-one (8):

A solution of the diol **7** (6.0 g, 21.2 mmol) and dihydropyran (7.80 ml, 84.8 mmol) in CH₂Cl₂ (100 ml) was cooled to 0°C. A catalytic amount of *p*-TsOH (0.05 g, 0.26 mmol) was added and the mixture was stirred for 30 min at 0°C. The reaction was then quenched by adding sat. aqueous NaHCO₃ (10 ml). The layers were separated and the aqueous phase was extracted with 2 X 25 ml of CH₂Cl₂. The combined organic layers were dried over anhydrous K₂CO₃, filtered and concentrated to afford a colorless oil which was purified by passage through a short column of silica (R_{*f*} =0.46, 1:1 EtOAc/hexanes). The bis-THP ether **8** (8.59 g, 89% yield) was isolated as a colorless oil which solidified upon standing. ¹H NMR (CDCl₃) δ (characteristic peaks only) 5.00 (m, 1H), 4.75 - 4.45 (m, 2H), 3.85 (m, 2H), 3.60 - 3.30 (m, 4H).

### F: (9S, 11R, 15R)-11,15-Bis-(tetrahydropyran-2-yloxy)-15-cyclohexyl-2,3,4,5,6,16,17,18,19,20-decanor-9-(triethylsilyloxy)prostanol Triethylsilyl Ether (10):

A suspension of lithium aluminum hydride (1.43 g, 38.0 mmol) in 50 ml of anhydrous THF was cooled to 0°C and a solution of the lactone **8** (8.59 g, 19.0 mmol) in THF (100 ml) was added dropwise. The resulting mixture was stirred at 0°C for 3 h, after which 1.5 ml of H₂O, 1.5 ml of 15% NaOH and 4.5 ml of H₂O were sequentially added. After warming to ambient temperature, 100 ml of EtOAc was added and the solids were filtered off. The filter cake was washed thoroughly with 3 X 50 ml of EtOAc and the filtrates were dried by passage through a short pad of anhydrous MgSO₄. Evaporation afforded **9** (9.02 g) as a colorless oil which was used in the subsequent step without further purification (R_{*f*} =0.31, 80:20 EtOAc/hexanes).

A mixture of the crude diol **9** (9.02 g, 19.0 mmol), triethylsilyl chloride (9.65 ml, 57.0 mmol), dimethylaminopyridine (0.41 g, 3.42 mmol), triethylamine (16.0 ml 114 mmol) and anhydrous *N,N*-dimethylformamide (50 ml) was stirred at ambient temperature for 14 h under N₂. The reaction mixture was then diluted with 250 ml of CH₂Cl₂ and the solution was washed with 3 X 50 ml of H₂O. The combined water washes were extracted with 2 X 50 ml of CH₂Cl₂. The organic layers were combined, dried (MgSO₄), filtered and concentrated to afford a yellow oil which was chromatographed on silica (R_{*f*} =0.4, 1:9 EtOAc/hexanes). Pure **10** (11.23 g, 86% yield from **8**) was obtained as a slightly yellow oil. ¹H NMR (CDCl₃) δ (characteristic peaks only) 4.62 (m, 2H), 4.15 - 3.25 (broad m, 7H), 2.30 - 1.15 (broad m, 18H), 0.95 (broad t, 18H), 0.65 (broad q, 12H).

### G: (9S, 11R, 15R)-11,15-Bis-(tetrahydropyran-2-yloxy)-15-cyclohexyl-2,3,4,5,6,16,17,18,19,20-decanor-9-(triethylsilyloxy)prostanal (11):

A solution of oxalyl chloride (0.51 ml, 0.57 mmol) in anhydrous CH₂Cl₂ (15 ml) was cooled to -78°C under N₂. A solution of anhydrous DMSO (0.81 ml, 11.4 mmol) in CH₂Cl₂ (2.0 ml) was then added dropwise. After 2 min, a solution of **10** (2.6 g, 3.8 mmol) in 8 ml of dry CH₂Cl₂ was introduced dropwise via syringe over a period of 2 min. The resulting mixture was stirred at -78°C for 2 h at which time triethylamine (2.7 ml, 19.0 mmol) was added. The reaction was stirred for 15 min and then allowed to warm to ambient temperature. The mixture was partitioned between 100 ml of EtOAc and 10 ml of H₂O and the organic layer was washed with an additional 10 ml of H₂O, 10 ml of brine and dried (MgSO₄). Solvent removal gave a yellow oil which was subjected to chromatography on silica gel (R_{f} 0.2, 10% EtOAc/hexanes) to afford **11** (1.4 g, 65% yield) and some starting material (0.83 g). ¹H NMR (CDCl₃) δ 9.80 (broad s, 1H), 4.62 (m, 2H), 4.20 (m, 1H), 3.85 - 3.60 (m, 3H), 3.40 (m, 3H), 2.80 (m, 1H), 2.45 - 2.05 (m, 4H), 1.95 - 1.10 (broad m, 27H), 0.95 (broad t, 9H), 0.55 (broad q, 6H).

### H: (5Z)-(9S, 11R, 15R)-11,15-Bis-(tetrahydropyran-2-yloxy)-15-cyclohexyl-2,3,4,16,17,18,19,20-octanor-9-(triethylsilyloxy)-5-prostenoic Acid Methyl Ester (12):

A solution of 18-crown-6 (8.50 g, 32.1 mmol) and bis(2,2,2-trifluoroethyl) (methoxycarbonylmethyl)phosphonate (3.72 g, 11.7 mmol) in 110 ml of THF was cooled to -78°C. KHMDS (0.5 *M* in toluene, 23.4 ml, 11.7 mmol) was added to the above mixture and the solution was stirred for 15 min. Aldehyde **11** (6.11 g, 10.7 mmol) in 5.0 ml of THF was added dropwise over a period of 15 min. The reaction was stirred at -78°C for 2 h, then warmed up to 0°C and stirred at that temperature for 2 more hours. The reaction was quenched by adding 50 ml of saturated aqueous NH₄Cl and the mixture was allowed to warm to room temperature. The layers were separated and the aqueous layer was extracted with 2 X 50 ml of EtOAc. The combined organic layers were washed with 2 X 50 ml of brine and dried (K₂CO₃). Filtration and solvent removal gave a crude yellow oil which was purified by passage through a short plug of silica to afford a mixture of **12** and its E isomer (9:1 ratio, 6.28 g, 95% yield) The isomers were separated by chromatography on silica gel (R_{*f*} =0.56, and 0.47, for the major and minor isomers respectively, 40% Et₂O/hexane); 4.57 g of pure **12** and 0.97 g of a 1:1 *E*/*Z* mixture were isolated. ¹H NMR (CDCl₃) δ 6.35 (m, 1H), 5.78 (broad d, J = 12.0 Hz, 1H), 4.65 (m, 2H), 4.28 (m, 1H), 3.90 (m, 2H), 3.70 (s, 3H), 3.55-3.30 (m, 3H), 2.80 (m, 2H), 2.35-2.05 (m, 1H), 2.00-1.10 (broad m, 30H), 0.95 (broad t, 9H), 0.60 (broad q, 6H).

### I: (5Z)-(9S, 11R, 15R)-11,15-Bis-(tetrahydropyran-2-yloxy)-15-cyclohexyl-2,3,4,16,17,18,19,20-octanor-9-(triethylsilyloxy)-5-prosten-1-ol (13):

A solution of **12** (2.0 g, 3.22 mmol) in 20 ml of anhydrous THF was cooled to 0°C under N₂. A solution of diisobutylaluminum hydride (1.5 M in toluene, 6.5 ml, 9.66 mmol) was added dropwise and the resulting mixture was stirred at 0°C for 2 h. The reaction was then quenched by careful addition of CH₃OH (5 ml), allowed to warm up to ambient temperature, and diluted with 50 ml of THF. The resulting cloudy solution was treated with 50 ml of a saturated aqueous solution of sodium potassium tartrate and the biphasic mixture was stirred for 1 h. The layers were then separated and the aqueous layer was extracted with 2 X 50 ml of THF. The organic extracts were combined, washed with brine (50 ml), and dried (MgSO₄). Filtration and solvent removal gave a pale yellow oil which was purified by chromatography on silica gel (R_{*f*} =0.26, 4:6 Et₂O/hexane) to yield 13 (1.95 g, 95% yield) as a colorless oil. This compound was used immediately in the subsequent reaction. ¹H NMR (CDCl₃) δ 5.65 (m, 2H), 4.65 (m, 2H), 4.30-3.25 (broad m, 5H), 2.40-2.05 (broad m, 4H), 2.00-1.10 (broad m, 32H), 1.00 (broad t, 9H), 0.60 (broad q, 6H).

### I: (5Z)-(9S, 11R, 15R)-11,15-Bis-(tetrahydropyran-2-yloxy)-15-cyclohexyl-9-hydroxy-3-oxa-16,17,18,19,20-pentanor-5-prostenoic Acid t-Butyl Ester (15):

A mixture of **13** (1.95 g, 3.28 mmol), *t*-butyl bromoacetate (5.11 g, 26.24 mmol), tetrabutylammonium hydrogen sulfate (0.8 g, 2.35 mmol), toluene (45 ml) and 25% (w/w) aqueous NaOH (30 ml) was stirred vigorously at room temperature for 18 h. The layers were separated and the aqueous layer was extracted with 2 X 25 ml of EtOAc. The combined organic extracts were washed with brine (15 ml), dried (MgSO₄), and concentrated. The crude product was purified by chromatography on silica gel (R_{*f*}=0.56, 20% EtOAc/hexane) to yield 2.19 g of **14** (contaminated with some *t*-butyl bromoacetate) and 0.48 g of the starting allyl alcohol 13. The allyl ether **14** thus obtained was used in the desilylation reaction without further purification.

The silyl ether **14** (0.5 g) obtained above was dissolved in 3.0 ml of DMSO and to it was added 2.2 ml of tetrabutylammonium fluoride (1.0 M in THF, 2.2 mmol). The mixture was stirred at ambient temperature for 30 min and then partitioned between 50 ml EtOAc and 10 ml of brine. The aqueous layer was extracted with 2 X 10 ml of EtOAc and the combined organic extracts were dried over MgSO₄. Evaporation and chromatography on silica gel (R_{*f*} =0.44, 50% EtOAc/hexane) afforded 0.28 g of **15** as a colorless oil. ¹H NMR (CDCl₃) δ 5.65 (m, 2H), 4.62 (m, 2H), 4.16 (m,. 1H), 4.10-3.75 (m, 3H), 3.95 (s, 2H), 3.45 (m, 2H), 2.50-0.90 (broad m, 35H), 1.46 (s, 9H); High Resolution CI MS *m*/*z* (CI) calcd. for C₃₄H₅₉O₈ (MH⁺) 595.4209, found 595.4208.

### K: (5Z)-(9R, 11R, 15R)-9-Chloro-15-cyclohexyl-11,15-dihydroxy-3-oxa-16,17,18,19,20-pentanor-5-prostenoic Acid t-Butyl Ester (V):

The hydroxyester **15** (0.28 g, 0.47 mmol) was dissolved in 4.0 ml of a stock solution containing 48.0 ml of CH₃CN, 0.79 ml of pyridine, and 0.97 ml of CCl₄. Triphenylphosphine (0.18 g, 0.70 mmol) was added and the resulting mixture was stirred at ambient temperature for 17 h. The reaction mixture was treated with 10 ml of a 1:1 solution of Et₂O/hexanes and the precipitate formed was filtered off. The filtrate was concentrated and purified by chromatography (silica gel, R_{*f*} =0.47, 40:60 Et₂O/hexanes) to yield pure **16** (90 mg, 34%) as a colorless oil.

A solution of **16** (80 mg, 0.13 mmol) in 7.0 ml of 65% (v/v) aqueous acetic acid was heated to 65-70°C for 45 min. The reacton mixture was cooled to room temperature and concentrated. The resulting residue was redissolved in anhydrous EtOH and the solvent was again evaporated. The residue thus obtained was purified by chromatography on silica gel (R_{*f*} =0.4, 60:40 EtOAc/hexanes) to yield 60 mg (100%) of **V** as a colorless, viscous oil. ¹H NMR (CDCl₃) δ 5.69 (m, 2H), 4.32 - 3.85 (m, 5H), 3.38 (m, 1H), 2.50 - 1.95 (m, 5H), 1.95 - 0.80 (broad m, 29H) 1.43 (s, 9H); ¹³C NMR (CDCl₃) δ 169.9, 131.7, 126.8, 82.0, 75.6, 75.1, 67.9, 66.6, 54.2, 51.0, 44.3, 43.7, 31.4, 30.3, 30.1 29.3, 28.1, 28.0, 26.5, 26.3, 26.1; High Resolution Cl MS *m*/*z* calcd. for C₂₄H₄₂O₅Cl (MH⁺) 445.2720, found 445.2716.

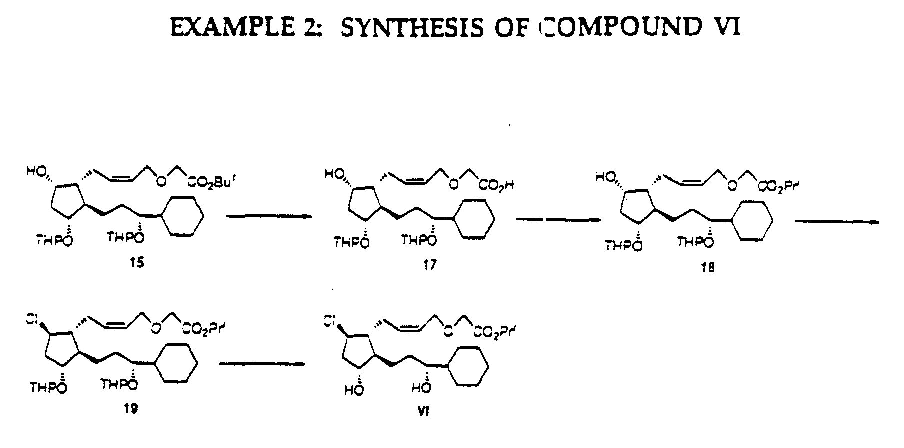

### A: (5Z)-(9S, 11R, 15R)-11,15-Bis-(tetrahydropyran-2-yloxy)-15-cyclohexyl-9-hvdroxy-3-oxa-16,17,18,19,20-pentanor-5-prostenoic Acid (17):

Hydroxyester **15** (0.454 g; 0.76 mmol; see Example 1) was dissolved in 10 ml of methanol and 2 ml of water. Lithium hydroxide monohydrate (0.16 g; 500 mol%) was added and the mixture was stirred at room temperature. After 18 h, 20 ml of saturated, aqueous KH₂PO₄ and 20 ml CH₂Cl₂ were added, the layers were separated, and the aqueous phase was washed with additional CH₂Cl₂ (3 X 20 ml). The combined organic layers were dried over Na₂SO₄, filtered, and concentrated, affording 0.47 g of a colorless oil which was used directly in the next reaction.

### B: (5Z)-(9S, 11R, 15R)-11,15-Bis-(tetrahydropyran-2-yloxy)-15-cyclohexyl-9-hydroxy-3-oxa-16,17,18,19,20-pentanor-5-prostenoic Acid Isopropyl Ester (18):

Crude acid **17** from above (0.23 g; 0.43 mmol) was dissolved in 10 ml of acetone. DBU (0.25 ml; 400 mol%) and isopropyl iodide (0.21 g; 300 mol%) were added and the mixture was stirred for 12 h at room temperature. After evaporation, the residue was applied to a silica gel column and eluted with hexane/EtOAc, 1/1, to afford 0.157 g (63%) of isopropyl ester **18** as a colorless oil (R_{*f*} =0.49). ¹H NMR (CDCl₃) δ (characteristic peaks only) 5.80-5.52 (m, 2H), 5.15 (sep, 1H, *J*=6.2 Hz), 4.03 (broad s, 2H), 1.27 (d, 6H, *J*=6.2 Hz).

### C: (5Z)-(9R, 11R, 15R)-9-Chloro-15-cyclohexyl-11,15-dihydroxy-3-oxa-16,17,18,19,20-pentanor-5-prostenoic Acid Isopropyl Ester (VI):

The hydroxyester **18** (0.146 g; 0.25 mmol) was dissolved in 3.0 ml of a stock solution containing 48 ml of CH₃CN, 0.79 ml of pyridine, and 0.97 ml of CCl₄. Triphenylphosphine (0.10 g; 150 mol%) was added and the resulting mixture was stirred at room temperature for 17 h. The reaction mixture was treated with 10 ml of a 1:1 solution of Et₂O/hexanes and the precipitate was filtered off. The filtrate was concentrated and chromatographed on silica gel (hexane/EtOAc, 4/1), affording 0.108 g of a colorless oil which consisted of a nearly equimolar mixture of desired chlorinated material **19** with its undesired 5,8-diene elimination product.

A solution of crude **19** from above in 10 ml of 65% (v/v) aqueous acetic acid was warmed to 65°C for 45 min. The mixture was then cooled to room temperature and concentrated. The resulting residue was then purified by silica gel chromatography (hexane/EtOAc, 2/3), affording 27 mg (25% based on **18**) of pure **VI** (R_{*f*} *=*0.56) as a colorless oil, and 69 mg of a mixture of **VI** and its 5,8-diene elimination product (R_{*f*} =0.45). ¹H NMR (CDCl₃) δ 5.67 (m, 2H), 5.08 (sep, 1H, *J*=6.1), 4.30-3.95 (m, 6H), 3.40 (m, 1H), 2.35 (m, 2H), 2.30-2.00 (m, 3H), 1.93-1.35 (m, 12H), 1.25 (d, 6H, *J*=6.2 Hz), 1.22-0.90 (m, 6H); ¹³C NMR (CDCl₃) δ 170.2, 131.8, 126.7, 75.7, 75.2, 68.8, 67.6, 66.7, 61.2, 54.2, 51.1, 44.4, 43.6, 31.4, 30.2, 30.1, 29.3, 28.0, 26.5, 26.3, 26.1, 21.8; High Resolution CI MS *m*/*z* cilcd. for C₂₃ H₄₀O₅Cl (MH⁺) 431.2564. found 431.2569.

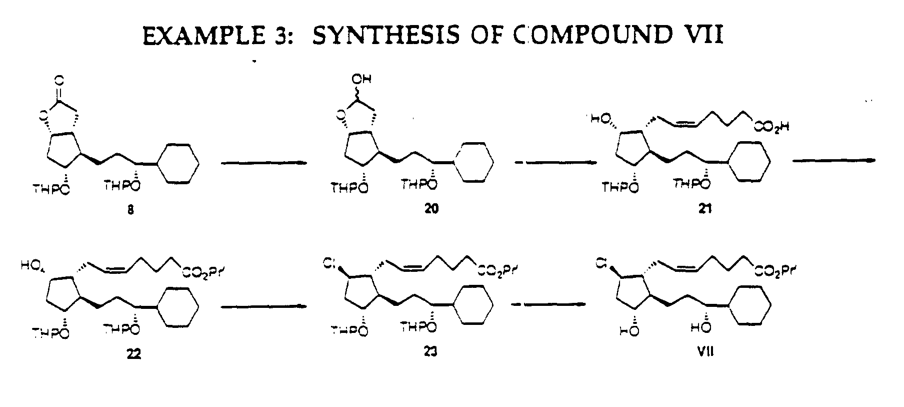

### A: (5Z)-(9S, 11R, 15R)-11,15-Bis-(tetrahydropyran-2-yloxy)-15-cyclohexyl-9-hydroxy-16,17,18,19,20-pentanor-5-prostenoic Acid Isopropyl Ester (22):

To a solution of 5.0 g (11 mmol) of lactone **8** (see Example 1) in 40 ml of THF at -78°C was added dropwise 9.6 ml (14.4 mmol) of a 1.5 *M* solution of diisobutylaluminum hydride in toluene. After 1.5 h, 5 ml of MeOH was added, the mixture was stirred for 10 min at -78°C and then warmed to room temperature. This solution was then added to a mixture of 20 ml of saturated, aqueous NH₄Cl, 35 ml of EtOAc, and 35 ml of saturated, aqueous sodium potassium tartrate. The mixture was stirred for 20 min, layers were separated, and the aqueous layer was washed with EtOAc (3 X 40 ml). The combined organic layers were dried over MgSO₄, filtered, and evaporated. The resulting residue was purified by silica gel chromatography (EtOAc/hexane, 1/1), affording 4.5 g (90%) of lactol **20** which was used directly in the next reaction.

To a solution of 14.1 g (31.8 mmol) of (1-carboxypent-5-yl)triphenylphosphonium bromide in 100 ml of THF at 0°C was added dropwise 59 ml (59 mmol) of a 1 *M* solution of potassium t-butoxide in THF. After 20 min, 4.5 g (9.9 mmol) of lactol **20** in 20 ml of THF was added cropwise. The reaction was quenched after 2 h by pouring into 150 ml of a 1/1 (v/v) mixture of EtOAc/saturated, aqueous NH₄Cl. The layers were separated and the aqueous layer was extracted with EtOAc (3 X 70 ml). The combined organic layers were dried over MgSO₄, filtered and evaporated, leaving 7.6 g of crude acid **21** as an oil.

Crude acid **21** (7.6 g) was dissolved in 55 mL of acetone, cooled to 0 °C, and 8.6 g (56 mmol) of DBU was added dropwise. The reaction was warmed to room temperature and 8.5 g (50 mmol) of isopropyl iodide was added dropwise. After stirring for 14 h, the mixture was poured into 100 ml of a 1/1 (v/v) mixture of EtOAc/saturated, aqueous NH₄Cl. The layers were separated and the aqueous phase was extracted with additional EtOAc (2 X 100 ml). The combined organic layers were dried over MgSO₄, filtered, evaporated, and chromatographed on silica gel (EtOAc/hexane, 2/3) affording 1.98 g (35% from lactol **20**) of **22**. ¹H NMR (CDCl₃) δ (characteristic peaks only) 5.58-5.28 (m 2H), 4.97 (sep, *J*=6.2 Hz, 1H), 1.1 (d, *J*=6.2 Hz, 6 H).

### B: (5Z)-(9R, 11R, 15R)-9-Chloro-15-cyclohexyl-11,15-dihydroxy-16,17,18,19,20-pentanor-5-prostenoic Acid Isopropyl Ester (VII):

Hydroxyester **22** (513.1 mg; 0.8864 mmol) was dissolved in 6.6 ml of a stock solution containing 48.0 ml of CH₃CN, 0.79 ml of pyridine, and 0.97 ml of CCl₄. Triphenylphosphine (348.8 mg; 150 mol%) was added and the mixture was stirred for 45 h at room temperature. The reaction was then diluted with 7 ml of Et₂O and 14 ml of hexane. After stirring for 10 min, solids were filtered off and the filtrate was evaporated. The resulting solids were triturated three times with 15 mL of hexane/Et₂O (1/2). Combined hexane/Et₂O washes were concentrated down to 0.75 g of a white solid which was then redissolved in hexane/Et₂O and chromatographed on silica gel. Elution with hexane/Et₂O, 5/1, afforded 372.8 mg of semipure **23** which was used directly in the next reaction.

Crude **23** from above was dissolved in 20 ml of 65% (v/v) aqueous HOAc and warmed to room temperature. After 1.5 h, the reaction was concentrated, 15 ml of H₂O was added, and the solution was reconcentrated. Absolute EtOH (15 ml) was added followed, again, by reconcentration. The resulting oil was purified by silica gel chromatography (hexane/EtOAc, 2/1), affording 244.1 mg of a mixture of **VII** contaminated with an approximately equal quantity of the 5,8-diene side product. An 8.2 mg sample was then further purified by reverse phase HPLC, giving 4.4 mg of pure **VII** as a clear, viscous oil. ¹H NMR (CDCl₃) δ 5.47 (m, *J*=8.5 Hz, 2H), 5.01 (sep, *J*=6.3Hz, 1H), 4.10 (dt, *J*=4.0, 6.2 Hz, 1H), 4.04 (q, *J*=7.6 Hz, 1H), 3.37 (m, 1H), 2.35-2.24 (m, 4H), 2.2C-2.07 (m, 4 H), 1.82 (br s, 2H), 1.80-1.50 (m, 13H), 1.36-0.96 (m, 12H). ¹³C NMR (CDCl₃) δ 173.2, 131.0, 126.8, 76.2, 76.0, 67.5, 60.8, 54.3, 51.7, 44.5, 43.5, 34.0, 31.7, 30.0, 29.2 (two overlapping resonances), 27.9, 26.6, 26.4, 26.2, 26.1, 24.9, 21.8. High Resolution CI MS *m*/*z* calcd. for C₂₄H₄₂O₄Cl (MH⁺) 429.2772, found 429.2763.

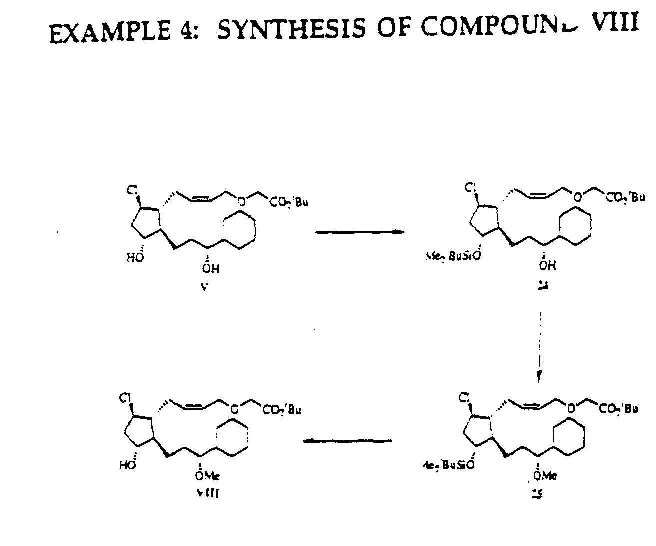

### A: (5Z)-(9R, 11R, 15R)-11-(t-Butyldimethylsiloxy)-9-chloro-15-cyclohexyl-15-hydroxy-3-oxa-16,17,18,19,20-pentanor-5-prostenoic acid t-butyl ester (24):

To a mixture of 127 mg (0.285 mmol) of Compound **V** (Example 1), 49 mg (0.72 mmol) of imidazole, 10 mg (0.082 mmol) of 4-(dimethylamino)pyridine (DMAP), and 5 ml of CH₂Cl₂ was added 90 mg (0.59 mmol) of *tert*butyldimethylsilyl chloride. After stirring overnight, 10 ml of saturated NH₄Cl was added, the mixture was extracted with CH₂Cl₂ (3 X 10 ml), the combined organic layers were dried over MgSO₄, filtered and concentrated, and the residue was chromatographed on silica gel (20% ethyl acetate in hexane) to afford 87 mg (55%) of **24.**

### B: (5Z)-(9R, 11R, 15R)-11-(t-Butyldimethylsiloxy)-9-chloro-15-cyclohexyl-15-methoxy-3-oxa-16,17,18,19,20-pentanor-5-prostenoic add t-butyl ester (25):

A mixture of 80 mg (0.14 mmol) of **24,** 100 mg (0.52 mmol) of 2,6-di-*t*-butylpyridine, 80 mg (0.51 mmol) of methyl trifluoromethanesulfonate, and 2 ml of CH₂Cl₂ was refluxed overnight. The reaction was cooled to room temperature, poured into 10 mL of saturated NaHCO₃, extracted with CH₂Cl₂ (3 X 10 ml), the combined organic layers were dried over MgSO₄, filtered, and concentrated, and the residue was chromatographed on silica gel (10% ethyl acetate in hexane) to afford 35 mg (44%) of **25.**

### C: (5Z)-(9R, 11R, 15R)-9-Chloro-15-cyclohexyl-11-hydroxy-15-methoxy-3-oxa-16,17,18,19,20-pentanor-5-prostenoic acid t-butyl ester (VIII):

To a mixture of 32 mg (0.056 mmol) of 25 and 1.5 mL of THF was added 0.12 ml (0.12 mmol) of 1 *M* tetrabutylammonium fluoride (TBAF) in THF. After 30 min, 4 ml of saturated NH₄Cl was added, the mixture was extracted with ethyl acetate (3 X 5 ml), the combined organic layers were dried over MgSO₄, filtered, and concentrated, and the residue was chromatographed on silica gel (40% ethyl acetate in hexane) to afford 24 mg (93%) of **VIII**. ¹³C NMR (CDCl₃) δ 169.77 (C), 130.90 (CH), 127.43 (CH), 85.89 (CH), 81.69 (C), 76.05 (CH), 67.83 (CH₂), 66.56 (CH₂), 61.00 (CH), 57.83 (CH3), 54.06 (CH), 51.92 (CH), 44.45 (CH₂), 40.65 (CH), 29.92 (CH₂), 29.83 (CH₂), 29.02 (CH₂), 28.42 (CH₃), 28.10 (CH₂), 26.64 (CH₂), 26.37 (CH₂). CI MS, *m*/*z* calcd. for C₂₅H₄₄O₅Cl (MH⁺) 459.2877, found 459.2877.

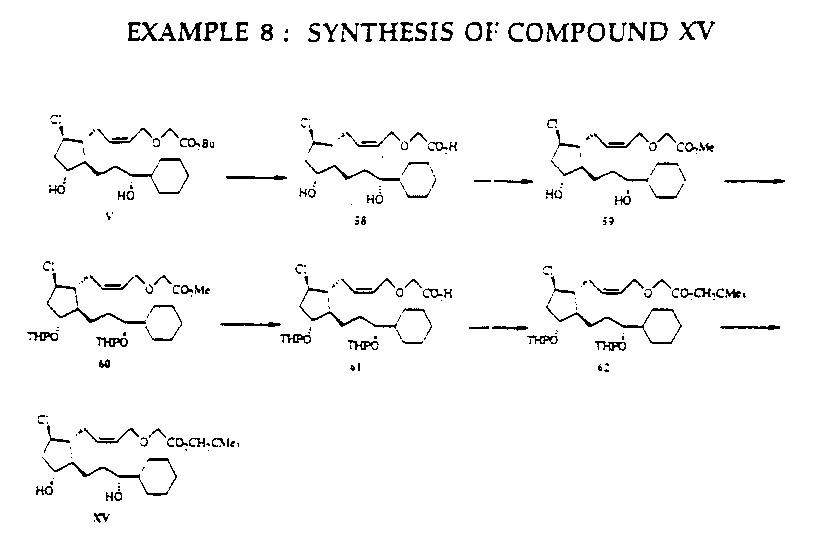

### A: (5Z)-(9R, 11R, 15R)-9-Chloro-15-cyclohexyl-11,15-dihydroxy-3-oxa-16,17,18,19,20-pentanor-5-prostenoic acid (58):

To a solution of 32 mg (0.072 mmol) of V (Example 1) in a mixture or 2.5 ml of methanol and 1.2 ml of water was added 15 mg (0.36 mmol) of lithium hydroxide monohydrate. After 9 h. 5 ml of 0.1 *M* HCl was added, and the mixture was extracted with CHCl₃ (5 X 5 ml). The combined organic layers were washed with water (20 ml) and brine (20 ml), dried over Na₂SO₄, filtered, and concentrated to afford 30 mg (100%) of 58 as a colorless oil. ¹³C NMR (CDCl₃) δ 173.17 (C), 132.77 (CH), 126.03 (CH), 75.68 (CH), 75.24 (CH), 66.89 (CH₂), 66.40 (CH₂), 61.32 (CH), 54.12 (CH), 50.62 (CH), 43.59 (CH₂), 43.33 (CH), 30.92 (CH₂), 30.73 (CH₂), 29.89 (CH₂), 29.30 (CH₂), 27.95 (CH₂), 26.44 (CH₂), 26.25 (CH₂), 26.09 (CH₂).

### B:(5Z)-(9R, 11R, 15R)-9-Chloro-15-cyclohexyl-11,15-dihydroxy-3-oxa-16,17,18,19,20-pentanor-5-prostenoic acid methyl ester (59):

To a solution of **58** (22 mg, 0.057 mmol) in 20 ml of ether was added excess diazomethane as a solution in ether. After 20 min residual diazomethane was removed from solution by a stream of N₂ and the solution was concentrated. The residue was chromatographed on silica gel (40% ethyl acetate in hexane) to afford **59** (21 mg, 91%). ¹³C NMR (CDCl₃) δ 171.06 (C), 131.75 (CH), 126.74 (CH), 75.73 (CH), 75.47 (CH), 67.34 (CH₂), 66.77 (CH₂), 61.08 (CH), 54.19 (CH), 51.94 (CH₃), 51.29 (CH), 44.47 (CH₂), 43.62 (CH), 31.49 (CH₂), 30.03 (CH₂), 29.30 (CH₂), 27.98 (CH₂), 26.49 (CH₂), 26.28 (CH₂), 26.13 (CH₂).

### C: (5Z)-(9R, 11R, 15R)-11,15-Bis(tetrahydropyran-2-yloxy)-9-chloro-15-cyclohexyl-3-oxa-16,17,18,19,20-pentanor-5-prostenoic acid methyl ester (60):

To a mixture of diol **59** (645 mg, 0.65 mmol). DHP (0.56 mL, 6.4 mmol), and CH₂Cl₂ (10 ml) at 0°C was added pTSA (10 mg, 0.053 mmol). After 15 min, 15 mL of saturated NaHCO₃ was added, the layers were separated, the aqueous layer was extracted with CH₂Cl₂ (3 X 15 ml), the combined organic layers were dried over MgSO₄, filtered, and concentrated, and the residue was chromatographed on silica gel (30% ethyl acetate in hexane) to afford 781 mg (86%) of bis-THP ether **60.**

### D: (5Z)-(9R, 11R, 15R)-11.15-Bis(tetrahydropyran-2-yloxy)-9-chloro-15-cyclohexyl-3-oxa-16,17,18,19,20-pentanor-5-prostenoic acid (61):

A solution of **60** (342 mg, 0.65 mmol) and lithium hydroxide monohydrate (96 mg, 2.3 mmol) in methanol (20 ml) was stirred for 2 h, the pH of the solution was adjusted to pH 6 with saturated KH₂PO₄, and he mixture was extracted with CH₂Cl₂ (6 X 20 ml). The combined organic layers were dried over Na₂SO₄, filtered, and concentrated to afford acid **61** (334 mg, 92%).

### E: (5Z)-(9R, 11R, 15R)-11.15-Bis(tetrahydropyran-2-yloxy)-9-chloro-15-cyclohexyl-3-oxa-16,17,18,19,20-pentanor-5-prostenoic add neopentyl ester (62):

A mixture of **61** (80 mg, 0.14 mmol), dicyclohexylcarbodiimide (30 mg, 0.18 mmol), DMAP (8 mg, 0.07 mmol), 2,2-dimethyl-1-propanol (60 mg, 0.7 mmol), and CH₂Cl₂ (1 ml) was stirred for 3 h at room temperature, the solution was concentrated, and the residue was chromatographed on silica gel (20% ethyl acetate in hexane) to provide **62** contaminated with some dicyclohexyl urea (110 mg total). The sample was used in the next reaction without further purification.

### F: (5Z)-(9R, 11R, 15R)-9-Chloro-15-cyclohexyl-11,15-dihydroxy-3-oxa-16,17,18,19,20-pentanor-5-prostenoic acid neopentyl ester (XV):

The 110 mg sample from above was dissolved in a mixture of methanol (10 ml) and water (1 ml) at 0°C, and 10 drops of 12 *N* HCl was added. After 15 min, the solution was warmed to room temperature for 30 min. The solution was added to CH₂Cl₂ (10 ml), the layers were separated, and the aqueous layer was extracted with CH₂Cl₂ (3 X 10 ml). The combined organic layers were dried over MgSO₄, filtered, concentrated, and chromatographed on silica gel (1/1 ethyl acetate/hexane) to afford **XV** (46 mg, 71% from **61**). ¹³C NMR (CDCl₃) δ 170.88 (C), 131.82 (CH), 126.72 (CH), 75.68 (CH), 75.23 (CH), 74.20 (CH₂), 67.34 (CH₂) 66.76 (CH₂), 61.23 (CH), 54.24 (CH), 51.19 (CH), 44.43 (CH₂), 43.65 (CH), 31.44 (CH₂), 31.34 (C), 30.23 (CH₂), 30.09 (CH₂), 29.33 (CH₂), 28.00 (CH₂), 26.50 (CH₂), 26.37 (CH₃), 26.28 (CH₂), 26.14 (CH₂). CI MS *m*/*z* calcd. for C₂₅H₄₄O₅Cl (MH⁺) 459.2877, found 459.2872. The compounds of formula (I) are useful in lowering intraocular pressure and thus are useful in the treatment of glaucoma. The preferred route of administration is topical. The dosage range for topical administration is generally between about 0.001 and about 1000 micrograms per eye (µg/eye) and is preferably between about 0.01 and about 100 µg/eye and most preferably between about 0.05 and 50 µg/eye. The compounds of the present invention can be administered as solutions, suspensions, or emulsions (dispersions) in a suitable ophthalmic vehicle.

In forming compositions for topical administration, the compounds of the present invention are generally formulated as between about 0.00002 to about 0.5 percent by weight (wt%) solutions in water at a pH between about 4.5 and about 8.0. The compounds are preferably formulated as between about 0.0001 to about 0.1 wt% and, most preferably, between about 0.001 and about 0.05 wt%. While the precise regimen is left to the discretion of the clinician, it is recommended that the compositions be topically applied by placing one or more drops in each eve one or more times a day.

Other ingredients which may be desirable to use in the ophthalmic preparations of the present invention include preservatives, co-solvents and viscosity building agents.

### Antimicrobial Preservatives:

Ophthalmic products are typically packaged in multidose form, which generally require the addition of preservatives to prevent microbial contamination during use. Suitable preservatives include: benzalkonium chloride, thimerosal, chlorobutanol, methyl paraben, propyl paraben, phenylethyl alcohol, edetate disodium, sorbic acid, ONAMER M®, or other agents known to those skilled in the art. Such preservatives are typically employed at a concentration between about 0.001 and about 1.0 wt%.

### Co-Solyents:

Prostaglandins, and particularly ester derivatives, typically have limited solubility in water and therefore may require a surfactant or other appropriate cosolvent in the composition. Such co-solvents include: Polysorbate 20 60 and 80; Pluronic F-68, F-84 and P-103 (trademarks); Tyloxapol (trademarks); Cremophor EL (trademarks); sodium dodecyl sulfate; glycerol; PEG 400; propylene glycol; cyclodextrins; or other agents known to those skilled in the art. Such co-solvents are typically employed at a concentration between about 0.01 and about 2 wt%.

### Viscosity Agents:

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include: polyvinyl alcohol; polyvinyl pyrrolidone; cellulosic polymers, such as methyl cellulose, hydroxy propyl methylcellulose, hydroxyethyl cellulose, carboxymethyl cellulose, hydroxy propyl cellulose; carboxy vinyl polymers, such as carbomer 910, carbomer 940, carbomer 934P and carbomer 1342; or other agents known to those skilled in the art. Such agents are typically used at a concentration between about 0.01 and about 2 wt%.

### EXAMPLE 9

The following Tables 2-4 containing Formulations A-M are representative pharmaceutical compositions of the invention for topical use in lowering of intraocular pressure. Each of Formulations A-M may be formulated in accordance with procedures known to those skilled in the art.

**TABLE 2**

| | **FORMULATION (wt%)** | | |
|---|---|---|---|
| **INGREDIENT** | **A** | **B** | **C** |
| Dextran 70 | 0.1 | 0.1 | -- |
| Hydroxypropyl Methylcellulose | 0.3 | 0.5 | --- |
| Monobasic Sodium Phosphate | --- | 0.05 | 0.05 |
| Dibasic Sodium Phosphate (anhydrous) | --- | 0.15 | 0.15 |
| Sodium Chloride | 0.77 | 0.75 | 0.5 |
| Potassium Chloride | 0.12 | --- | --- |
| Disodium EDTA | 0.05 | 0.05 | --- |
| Hydroxypropyl-β-cyclodextrin | --- | --- | 1.0 |
| Tyloxapol | --- | 0.1 | --- |
| Benzalkonium Chloride | 0.01 | 0.01 | 0.01 |
| Polysorbate 80 | 0.01 | --- | --- |
| HCl and/or NaOH | q.s. to pH 7.2-7.5 | q.s. to pH 7.3-7.4 | q.s. to pH 6.3-6.6 |
| Purified Water | q.s. to 100% | q.s. to 100% | q.s. to 100% |

**TABLE 3**

| | **FORMULATION (wt%)** | | | |
|---|---|---|---|---|
| **INGREDIENT** | **E** | **F** | **G** | **H** |
| Compound **V** | 0.01 | --- | --- | --- |
| Compound **VI** | --- | 0.01 | --- | --- |
| | | | | |
| Monobasic Sodium Phosphate | 0.05 | 0.05 | 0.05 | 0.05 |
| Dibasic Sodium Phosphate (anhydrous) | 0.15 | 0.15 | 0.15 | 0.15 |
| Sodium Chloride | 0.75 | 0.75 | 0.5 | 0.6 |
| Disodium EDTA | 0.01 | 0.05 | --- | --- |
| Cremophor® EL | --- | 0.1 | --- | --- |
| Hydroxypropyl-β-cyclodextrin | --- | --- | 4.0 | --- |
| Tyloxapol | --- | --- | --- | 0.5 |
| Benzalkonium Chloride | 0.02 | 0.01 | 0.01 | 0.02 |
| Polysorbate 80 | 0.05 | --- | --- | --- |
| HCl and/or NaOH | q.s. to pH 7.3-7.4 | q.s. to pH 7.3-7.4 | q.s. to pH 6.3-6.6 | q.s. to pH 6.3-6.6 |
| Purified Water | q.s. to 100% | q.s. to 100% | q.s. to 100% | q.s. to 100% |

**TABLE 4**

| | **FORMULATION (wt%)** | | | |
|---|---|---|---|---|
| **INGREDIENT** | **J** | **K** | **L** | **M** |
| Compound **VIII** | 0.01 | --- | --- | --- |
| | | | | |
| Monobasic Sodium Phosphate | 0.05 | 0.05 | 0.05 | 0.05 |
| Dibasic Sodium Phosphate (anhydrous) | 0.15 | 0.15 | 0.15 | 0.15 |
| Sodium Chloride | 0.75 | 0.75 | 0.5 | 0.6 |
| Disodium EDTA | 0.01 | 0.05 | --- | --- |
| Cremophor® EL | --- | 0.01 | --- | --- |
| Hydroxypropyl-β-cyclodextrin | --- | --- | 4.0 | --- |
| Tyloxapol | --- | --- | -- | 0.5 |
| Benzalkonium Chloride | 0.02 | 0.01 | 0.01 | 0.02 |
| Polvsorbate 80 | 0.05 | --- | --- | --- |
| HCl and/or NaOH | q.s. to pH 7.3-7.4 | q.s. to pH 7.3-7.4 | q.s. to pH 6.3-6.6 | q.s. to pH 6.3-6.6 |
| Purified Water | q.s. to 100% | q.s. to 100% | q.s. to 100% | q.s. to 100% |

### EXAMPLE 10

The ability of certain compounds of the present invention to reduce intraocular pressure (IOP) was evaluated in cynomolgus monkeys with ocular hypertension produced by previous laser trabeculoplasty in the right eye. Animals had been trained to sit in restraint chairs and conditioned to accept experimental procedures without chemical restraint. IOP was determined with a pneumatonometer after light corneal anesthesia with dilute proparacaine. The test protocol included a treatment regimen consisting of 5 divided doses administered over a period of 2 and 1/2 days. Doses 2-5 were given 8, 24, 32 and 48 hours after the initial dose. Baseline IOP values were determined prior to treatment with the test formulation, and then IOP was determined 16 hours after the fourth dose, and 2, 4 and 6 hours after the fifth dose. Prostaglandin doses are micrograms of compound contained in each treatment.

The tested compounds are the previously identified Compounds **V-VII.**

**TABLE 5**

| **Compound** | **PG Dose** | **Baseline IOP (mm Hg)** | **Percent IOP Reduction at Hours after Dose/Dose#** | | | |
|---|---|---|---|---|---|---|
| | | | **16/4** | **2/5** | **4/5** | **6/5** |
| **V** | 5 µg | 36.3 | 38.3±3.0 | 47.5±5.1 | 43.7±5.6 | 36.5±5.8 |
| **VI** | 1 µg | 36.9 | 22.3±2.7 | 30.8±4.7 | 26.4±4.7 | 24.9±4.0 |
| **VII** | 20 µg | 32.6 | 13.9±2.7 | 30.1±4.8 | 22.4±5.2 | 19.3±4.3 |

Results are presented in Table 5, above. Compounds **v**-**VII** produced significant reduction of intraocular pressure at doses which are marginal or ineffective for other prostaglandins in published clinical studies.

For example, Nakajima et al. (Graefe's Arch. Clin. Exp. Ophthalmol., 229:411-413 (1991)) reported that 50 µg of PGD₂ and 2.5 µg of BW245C (a PGD₂ analogue) reduced intraocular pressure in human eyes by 12% and 10%, respectively. Other studies (Woodward et al., Invest. Ophthalmol. Vis. Sci., 31:138-146 (1990)) reported for these reference compounds in rabbits describe a maximum IOP reduction of approximately 28% for 250 µg of PGD₂ and 22% for 25 µg of BW245C. These comparisons indicate the unexpected potency of Compounds **V**- **VII** in reducing intraocular pressure. No indications of inflammation were observed during these studies.

## Claims (Claims for the following Contracting State(s): AT, BE, CH, DE, DK, ES, FR, GB, GR, IT, LU, MC, NL, SE)

1. A topical ophthalmic composition for the treatment of glaucoma and ocular hypertension adapted only for topical ophthalmic use, to the exclusion of other topical uses or parental uses or oral uses, said composition comprising an ophthalmically acceptable vehicle and a therapeutically effective amount of a compound of formula: 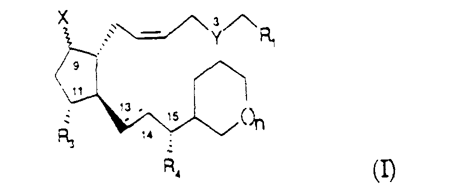 wherein
R₁ = CO₂R₂, wherein R₂ = H, a cationic salt moiety, or an ophthalmically acceptable ammonium moiety; or R₁ may also represent an ophthalmically acceptable ester moiety;
X = halogen, particularly Cl or F, in either configuration;
Y = CH₂ or O;
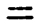 = single or *trans* double bond;
R₃, R₄ can be the same or different, and are selected from: free or functionally modified hydroxy groups; n = 0 or 1; and
() represents the fact that the ring may be 5-membered or 6-membered, with the proviso that formula I is not a compound of formula 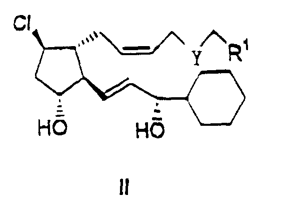 in which
Y = CH₂ or O; and
R₁ = COOR₂ in which R₂ = optionally substituted C₁₋₁₀ alkyl, C₃₋₁₀ cycloalkyl, C₆₋₁₀ aryl, or arylalkyl wherein the aryl portion contains
6-10 carbon atoms and the alkyl portion contains 1-4 carbon atoms.

2. The composition of claim 1, wherein: R₁ = CO₂R₂; R₂ = substituted or unsubstituted alkyl, cycloalkyl, (cycloalkyl)alkyl, aryl, arylalkyl, heteroaryl, or (heteroaryl)alkyl, wherein substituents are selected from the group consisting of: alkyl, halogen, a free or functionally modified hydroxy group, or a free or functionally modified thiol; and X = F or Cl in either configuration.

3. The composition of claim 2, wherein: R₁ = CO₂R₂; R₂ = H, methyl, ethyl, n-propyl, isopropyl, t-butyl or benzyl; X = Cl in the β (R) configuration; Y = O; R₃ and R₄ = OH; and n = 1.

4. The composition of claim 3, wherein R₁ = CO₂R₂ and R₂ = isopropyl.

5. The composition of claim 3, wherein: R₂ = t-butyl.

6. The composition of claim 2, wherein: R₁ = CO₂R₂; R₂ = H, methyl, ethyl, n-propyl, isopropyl, t-butyl, or benzyl; X = Cl in the β (R) configuration; Y = CH₂; R₃ and R₄ = OH; and n = 1.

7. The composition of claim 6, wherein: R₂ = methyl.

8. The composition of any of claims 1-6, wherein the compound of formula (I) is present at a concentration between about 0.00002 and about 0.5 percent by weight.

9. The composition of claim 8, wherein the compound of formula (I) is present at a concentration between about 0.0001 and about 0.1 percent by weight.

10. The composition of claim 9, wherein the compound of formula (I) is present at a concentration between about 0.001 and about 0.05 percent by weight.

11. Use of the composition of any of claims 1-6 for the manufacture of a medicament for the topical administration of said composition to a glaucomatous or hypertensive eye.

12. Use according to claim 11, wherein between about 0.001 and about 1000 micrograms of a compound of formula (I) is administered.

13. Use according to claim 12, wherein between about 0.01 and about 100 micrograms of a compound of formula (I) is administered.

14. Use according to claim 13, wherein between about 0.05 and about 50 micrograms of a compound of formula (I) is administered.

## Claims (Claims for the following Contracting State(s): IE, PT)

1. A topical ophthalmic composition for the treatment of glaucoma and ocular hypertension adapted only for topical ophthalmic use, to the exclusion of other topical uses or parental uses or oral uses, said composition comprising an ophthalmically acceptable vehicle and a therapeutically effective amount of a compound of formula: 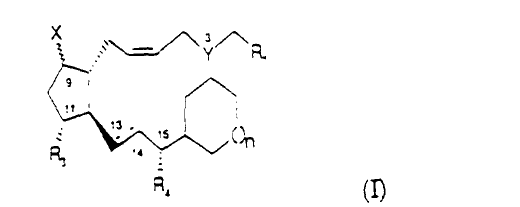 wherein
R₁ = CO₂R₂, wherein R₂ = H, a cationic salt moiety, or an ophthalmically acceptable ammonium moiety; or R₁ may also represent an ophthalmically acceptable ester moiety;
X = halogen, particularly Cl or F, in either configuration;
Y = CH₂ or O;
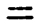 = single or *trans* double bond;
R₃, R₄ can be the same or different, and are selected from: free or functionally modified hydroxy groups;
n = 0 or 1; and
() represents the fact that the ring may be 5-membered or 6-membered.

2. The composition of claim 1, wherein: R₁ = CO₂R₂; R₂ = substituted or unsubstituted alkyl, cycloalkyl, (cycloalkyl)alkyl, aryl, arylalkyl, heteroaryl, or (heteroaryl)alkyl, wherein substituents are selected from the group consisting of: alkyl, halogen, a free or functionally modified hydroxy group, or a free or functionally modified thiol; and X = F or Cl in either configuration.

3. The composition of claim 2, wherein: R₁ = CO₂R₂; R₂ = H, methyl, ethyl, n-propyl, isopropyl, t-butyl or benzyl; X = Cl in the β (R) configuration; Y = O; R₃ and R₄ = OH; and n = 1.

4. The composition of claim 3, wherein R₁ = CO₂R₂ and R₂ = isopropyl.

5. The composition of claim 3, wherein: R₂ = t-butyl.

6. The composition of claim 2, wherein: R₁ = CO₂R₂; R₂ = H, methyl, ethyl, n-propyl, isopropyl, t-butyl, or benzyl; X = Cl in the β (R) configuration; Y = CH₂; R₃ and R₄ = OH; and n = 1.

7. The composition of claim 6, wherein: R₂ = methyl.

8. The composition of any of claims 1-6, wherein the compound of formula (I) is present at a concentration between about 0.00002 and about 0.5 percent by weight.

9. The composition of claim 8, wherein the compound of formula (I) is present at a concentration between about 0.0001 and about 0.1 percent by weight.

10. The composition of claim 9, wherein the compound of formula (I) is present at a concentration between about 0.001 and about 0.05 percent by weight.

11. Use of the composition of any of claims 1-6 for the manufacture of a medicament for the topical administration of said composition to a glaucomatous or hypertensive eye.

12. Use according to claim 11, wherein between about 0.001 and about 1000 micrograms of a compound of formula (I) is administered.

13. Use according to claim 12, wherein between about 0.01 and about 100 micrograms of a compound of formula (I) is administered.

14. Use according to claim 13, wherein between about 0.05 and about 50 micrograms of a compound of formula (I) is administered.

## Patentansprüche (Patentansprüche für folgende(n) Vertragsstaat(en): AT, BE, CH, DE, DK, ES, FR, GB, GR, IT, LU, MC, NL, SE)

1. Topische ophthalmische Zubereitung für die Behandlung von Glaukom und okulärer Hypertonie, angepasst nur für die topische ophthalmische Verwendung, unter Ausschluss anderer topischer Verwendungen oder parenteraler Verwendungen oder oraler Verwendungen, wobei die Zubereitung einen ophthalmisch annehmbaren Träger und eine therapeutisch wirksame Menge einer Verbindung der Formel: 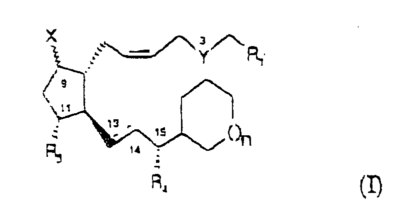 umfasst, worin:
R₁ CO₂R₂ bedeutet, worin R₂ H, eine kationische Salzgruppierung oder eine ophthalmisch annehmbare Ammoniumgruppierung bedeutet; oder worin R₁ auch eine ophthalmisch annehmbare Estergruppierung bedeuten kann;
X Halogen, insbesondere Cl oder F, in jeder Konfiguration bedeutet;
Y CH₂ oder O bedeutet;
eine Einfach- oder trans-Doppelbindung bedeutet;
R₃, R₄ gleich oder unterschiedlich sein können und ausgewählt sind aus freien oder funktionell modifizierten Hydroxygruppen;
n = 0 oder 1; und
() die Tatsache bedeutet, dass der Ring 5- oder 6-gliedrig sein kann, mit der Maßgabe, dass die Formel I nicht eine Verbindung der Formel 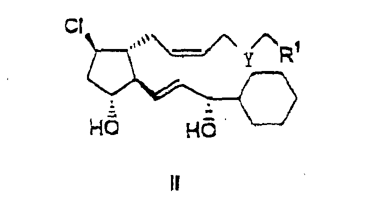 ist, worin
Y CH₂ oder O bedeutet; und
R₁ COOR₂ bedeutet, worin R₂ gegebenenfalls substituiertes C₁₋₁₀-Alkyl, C₃₋₁₀-Cycloalkyl, C₆₋₁₀-Aryl oder Aralkyl bedeutet; worin der Arylteil 6 bis 10 Kohlenstoffatome enthält und der Alkylteil 1 bis 4 Kohlenstoffatome enthält.

2. Zubereitung nach Anspruch 1, worin R₁ = CO₂R₂; R₂ = substituiertes oder unsubstituiertes Alkyl, Cycloalkyl, (Cycloalkyl)alkyl, Aryl, Arylalkyl, Heteroaryl oder (Heteroaryl)alkyl, worin die Substituenten ausgewählt sind aus der Gruppe bestehend aus Alkyl, Halogen, einer freien oder funktionell modifizierten Hydroxygruppe oder einem freien oder funktionell modifizierten Thiol; und X F oder Cl in jeder Konfiguration bedeutet.

3. Zubereitung nach Anspruch 2, worin R₁ = CO₂R₂; R₂ = H, Methyl, Ethyl, n-Propyl, Isopropyl, t-Butyl oder Benzyl; X = Cl in der β(R)-Konfiguration; Y = O; R₃ und R₄ = OH; und n = 1.

4. Zubereitung nach Anspruch 3, worin R₁ = CO₂R₂ und R₂ = Isopropyl.

5. Zubereitung nach Anspruch 3, worin R₂ = t-Butyl.

6. Zubereitung nach Anspruch 2, worin R₁ = CO₂R₂; R₂ = H, Methyl, Ethyl, n-Propyl, Isopropyl, t-Butyl oder Benzyl; X = Cl in der β(R)-Konfiguration; Y = CH₂; R₃ und R₄ = OH; und n = 1.

7. Zubereitung nach Anspruch 6, worin R₂ = Methyl.

8. Zubereitung nach einem der Ansprüche 1 bis 6, worin die Verbindung der Formel (I) in einer Konzentration zwischen etwa 0,00002 und etwa 0,5 Gew.-% vorhanden ist.

9. Zubereitung nach Anspruch 8, worin die Verbindung der Formel (I) in einer Konzentration zwischen etwa 0,0001 und etwa 0,1 Gew.-% vorhanden ist.

10. Zubereitung nach Anspruch 9, worin die Verbindung der Formel (I) in einer Konzentration zwischen etwa 0,001 und etwa 0,05 Gew.-% vorhanden ist.

11. Verwendung der Zubereitung nach einem der Ansprüche 1 bis 6 zur Herstellung eines Medikaments für die topische Anwendung der Zubereitung auf ein glaukomatöses oder hypertones Auge.

12. Verwendung nach Anspruch 11, wobei zwischen etwa 0,001 und etwa 1000 Mikrogramm einer Verbindung der Formel (I) verabreicht werden.

13. Verwendung nach Anspruch 12, wobei zwischen etwa 0,01 und etwa 100 Mikrogramm einer Verbindung der Formel (I) verabreicht werden.

14. Verwendung nach Anspruch 13, wobei zwischen etwa 0,05 und etwa 50 Mikrogramm einer Verbindung der Formel (I) verabreicht werden.

## Patentansprüche (Patentansprüche für folgende(n) Vertragsstaat(en): IE, PT)

1. Topische ophthalmische Zubereitung für die Behandlung von Glaukom und okulärer Hypertonie, angepasst nur für die topische ophthalmische Verwendung, unter Ausschluss anderer topischer Verwendungen oder parenteraler Verwendungen oder oraler Verwendungen, wobei die Zubereitung einen ophthalmisch annehmbaren Träger und eine therapeutisch wirksame Menge einer Verbindung der Formel: 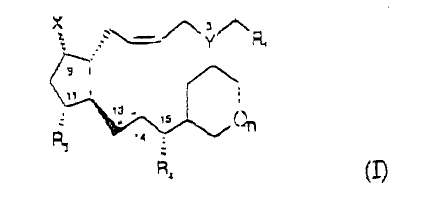 umfasst, worin:
R₁ CO₂R₂ bedeutet, worin R₂ H, eine kationische Salzgruppierung oder eine ophthalmisch annehmbare Ammoniumgruppierung bedeutet; oder worin R₁ auch eine ophthalmisch annehmbare Estergruppierung bedeuten kann;
X Halogen, insbesondere Cl oder F, in jeder Konfiguration bedeutet;
Y CH₂ oder O bedeutet;
eine Einfach- oder trans-Doppelbindung bedeutet;
R₃, R₄ gleich oder unterschiedlich sein können und ausgewählt sind aus freien oder funktionell modifizierten Hydroxygruppen;
n = 0 oder 1; und
() die Tatsache bedeutet, dass der Ring 5- oder 6-gliedrig sein kann.

2. Zubereitung nach Anspruch 1, worin R₁ = CO₂R₂; R₂ = substituiertes oder unsubstituiertes Alkyl, Cycloalkyl, (Cycloalkyl)alkyl, Aryl, Arylalkyl, Heteroaryl oder (Heteroaryl)alkyl, worin die Substituenten ausgewählt sind aus der Gruppe bestehend aus Alkyl, Halogen, einer freien oder funktionell modifizierten Hydroxygruppe oder einem freien oder funktionell modifizierten Thiol; und X F oder Cl in jeder Konfiguration bedeutet.

3. Zubereitung nach Anspruch 2, worin R₁ = CO₂R₂; R₂= H, Methyl, Ethyl, n-Propyl, Isopropyl, t-Butyl oder Benzyl; X = Cl in der β(R)-Konfiguration; Y = O; R₃ und R₄ = OH; und n = 1.

4. Zubereitung nach Anspruch 3, worin R₁ = CO₂R₂ und R₂ = Isopropyl.

5. Zubereitung nach Anspruch 3, worin R₂ = t-Butyl.

6. Zubereitung nach Anspruch 2, worin R₁ = CO₂R₂; R₂ = H, Methyl, Ethyl, n-Propyl, Isopropyl, t-Butyl oder Benzyl; X = Cl in der β(R)-Konfiguration; Y = CH₂; R₃ und R₄ = OH; und n = 1.

7. Zubereitung nach Anspruch 6, worin R₂ = Methyl.

8. Zubereitung nach einem der Ansprüche 1 bis 6, worin die Verbindung der Formel (I) in einer Konzentration zwischen etwa 0,00002 und etwa 0,5 Gew.-% vorhanden ist.

9. Zubereitung nach Anspruch 8, worin die Verbindung der Formel (I) in einer Konzentration zwischen etwa 0,0001 und etwa 0,1 Gew.-% vorhanden ist.

10. Zubereitung nach Anspruch 9, worin die Verbindung der Formel (I) in einer Konzentration zwischen etwa 0,001 und etwa 0,05 Gew.-% vorhanden ist.

11. Verwendung der Zubereitung nach einem der Ansprüche 1 bis 6 zur Herstellung eines Medikaments für die topische Anwendung der Zubereitung auf ein glaukomatöses oder hypertones Auge.

12. Verwendung nach Anspruch 11, wobei zwischen etwa 0,001 und etwa 1000 Mikrogramm einer Verbindung der Formel (I) verabreicht werden.

13. Verwendung nach Anspruch 12, wobei zwischen etwa 0,01 und etwa 100 Mikrogramm einer Verbindung der Formel (I) verabreicht werden.

14. Verwendung nach Anspruch 13, wobei zwischen etwa 0,05 und etwa 50 Mikrogramm einer Verbindung der Formel (I) verabreicht werden.

## Revendications (Revendications pour l'(les) Etat(s) contractant(s) suivant(s): AT, BE, CH, DE, DK, ES, FR, GB, GR, IT, LU, MC, NL, SE)

1. Composition ophtalmique à usage local pour le traitement du glaucome et de l'hypertension oculaire conçue uniquement pour une utilisation ophtalmique locale, à l'exclusion d'autres utilisations locales ou d'utilisations parentérales ou d'utilisations orales, ladite composition comprenant un véhicule ophtalmiquement acceptable et une quantité thérapeutiquement efficace d'un composé répondant à la formule 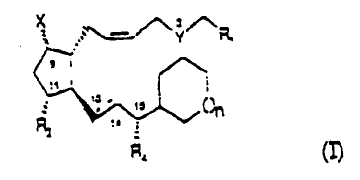 dans laquelle :
R₁ représente un groupe CO₂R₂ dans lequel R₂ représente un atome d'hydrogène, une fraction de sel cationique ou une fraction ammonium ophtalmiquement acceptable, ou bien R₁ peut également représenter une fraction ester ophtalmiquement acceptable ;
X représente un atome d'halogène, en particulier un atome de chlore ou un atome de fluor dans l'une ou l'autre configuration ;
Y représente un groupe CH₂ ou un atome d'oxygène ;
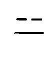 représente une liaison simple ou une liaison double *trans* ;
R₃, R₄ peuvent être identiques ou différents et sont choisis parmi le groupe comprenant des groupes hydroxyle libres ou modifiés pour les rendre fonctionnels ;
n est égal à 0 ou 1; et
0 représente le fait que le noyau peut être pentagonal ou hexagonal,
avec cette réserve que la formule I ne représente pas un composé répondant à la formule 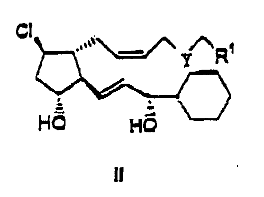 dans laquelle
Y représente un groupe CH₂ ou un atome d'oxygène ; et
R₁ représente un groupe COOR₂ dans lequel R₂ représente un groupe alkyle en C₁-C₁₀, un groupe cycloalkyle en C₃-C₁₀, un groupe aryle en C₆-C₁₀ ou un groupe arylalkyle, le cas échéant substitués, groupe arylalkyle dans lequel la portion aryle contient de 6 à 10 atomes de carbone et la portion alkyle contient de 1 à 4 atomes de carbone.

2. Composition selon la revendication 1, dans laquelle R₁ représente un groupe CO₂R₂ ; R₂ représente un groupe alkyle, un groupe cycloalkyle, un groupe (cycloalkyl)alkyle, un groupe aryle, un groupe arylalkyle, un groupe hétéroaryle ou un groupe (hétéroaryl)alkyle, chacun de ces groupes étant substitué ou non substitué, les substituants étant choisis parmi le groupe constitué par un groupe alkyle, un atome d'halogène, un groupe hydroxyle libre ou modifié pour le rendre fonctionnel ou encore un thiol libre ou modifié pour le rendre fonctionnel ; et X représente un atome d'halogène, en particulier un atome de chlore ou un atome de fluor dans l'une ou l'autre configuration.

3. Composition selon la revendication 2, dans laquelle R₁ représente un groupe CO₂R₂ ; R₂ représente un atome d'hydrogène, un groupe méthyle, un groupe éthyle, un groupe n-propyle, un groupe isopropyle, un groupe t-butyle ou un groupe benzyle ; X représente un atome de chlore dans la configuration β (R) ; Y représente un atome d'oxygène ; R₃ et R₄ représentent un groupe OH et n est égal à 1.

4. Composition selon la revendication 3, dans laquelle R₁ représente un groupe CO₂R₂ et R₂ représente un groupe isopropyle.

5. Composition selon la revendication 3, dans laquelle R₂ représente un groupe t-butyle.

6. Composition selon la revendication 2, dans laquelle R₁ représente un groupe CO₂R₂ ; R₂ représente un atome d'hydrogène, un groupe méthyle, un groupe éthyle, un groupe n-propyle, un groupe isopropyle, un groupe t-butyle ou un groupe benzyle ; X représente un atome de chlore dans la configuration β (R) ; Y représente un groupe CH₂ ; R₃ et R₄ représentent un groupe OH et n est égal à 1.

7. Composition selon la revendication 6, dans laquelle R₂ représente un groupe méthyle.

8. Composition selon l'une quelconque des revendications 1 à 6, dans laquelle le composé répondant à la formule (I) est présent en une concentration entre environ 0,00002 et environ 0,5 % en poids.

9. Composition selon la revendication 8, dans laquelle le composé répondant à la formule (I) est présent en une concentration entre environ 0,0001 et environ 0,1 % en poids.

10. Composition selon la revendication 9, dans laquelle le composé répondant à la formule (I) est présent en une concentration entre environ 0,001 et environ 0,05 % en poids.

11. Utilisation de la composition selon l'une quelconque des revendications 1 à 6 pour la préparation d'un médicament pour l'administration locale de ladite composition à un oeil glaucomateux ou hypersensible.

12. Utilisation selon la revendication 11, dans laquelle on administre le composé répondant à la formule (I) à concurrence d'environ 0,001 à environ 1000 microgrammes.

13. Utilisation selon la revendication 12, dans laquelle on administre le composé répondant à la formule (I) à concurrence d'environ 0,01 à environ 100 microgrammes.

14. Utilisation selon la revendication 13, dans laquelle on administre le composé répondant à la formule (I) à concurrence d'environ 0,05 à environ 50 microgrammes.

## Revendications (Revendications pour l'(les) Etat(s) contractant(s) suivant(s): IE, PT)

1. Composition ophtalmique à usage local pour le traitement du glaucome et de l'hypertension oculaire conçue uniquement pour une utilisation ophtalmique locale, à l'exclusion d'autres utilisations locales ou d'utilisations parentérales
ou d'utilisations orales, ladite composition comprenant un véhicule ophtalmiquement acceptable et une quantité thérapeutiquement efficace d'un composé répondant à la formule 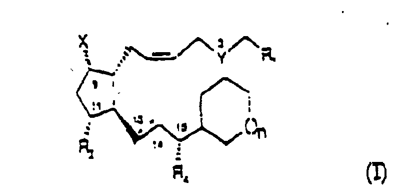 dans laquelle :
R₁ représente un groupe CO₂R₂ dans lequel R₂ représente un atome d'hydrogène, une fraction de sel cationique ou une fraction ammonium ophtalmiquement acceptable, ou bien R₁ peut également représenter une fraction ester ophtalmiquement acceptable ;
X représente un atome d'halogène, en particulier un atome de chlore ou un atome de fluor dans l'une ou l'autre configuration ;
Y représente un groupe CH₂ ou un atome d'oxygène ;
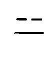 représente une liaison simple ou une liaison double *trans ;*
R₃, R₄ peuvent être identiques ou différents et sont choisis parmi le groupe comprenant des groupes hydroxyle libres ou modifiés pour les rendre fonctionnels ;
n est égal à 0 ou 1; et
0 représente le fait que le noyau peut être pentagonal
ou hexagonal.

2. Composition selon la revendication 1, dans laquelle R₁ représente un groupe CO₂R₂ ; R₂ représente un groupe alkyle, un groupe cycloalkyle, un groupe (cycloalkyl)alkyle, un groupe aryle, un groupe arylalkyle, un groupe hétéroaryle ou un groupe (hétéroaryl)alkyle, chacun de ces groupes étant substitué ou non substitué, les substituants étant choisis parmi le groupe constitué par un groupe alkyle, un atome d'halogène, un groupe hydroxyle libre ou modifié pour le rendre fonctionnel ou encore un thiol libre ou modifié pour le rendre fonctionnel ; et X représente un atome d'halogène, en particulier un atome de chlore ou un atome de fluor dans l'une ou l'autre configuration.

3. Composition selon la revendication 2, dans laquelle R₁ représente un groupe CO₂R₂ ; R₂ représente un atome d'hydrogène, un groupe méthyle, un groupe éthyle, un groupe nº propyle, un groupe isopropyle, un groupe t-butyle ou un groupe benzyle ; X représente un atome de chlore dans la configuration β (R) ; Y représente un atome d'oxygène ; R₃ et R₄ représentent un groupe OH et n est égal à 1.

4. Composition selon la revendication 3, dans laquelle R₁ représente un groupe CO₂R₂ et R₂ représente un groupe isopropyle.

5. Composition selon la revendication 3, dans laquelle R₂ représente un groupe t-butyle.

6. Composition selon la revendication 2, dans laquelle R₁ représente un groupe CO₂R₂ ; R₂ représente un atome d'hydrogène, un groupe méthyle, un groupe éthyle, un groupe n-propyle, un groupe isopropyle, un groupe t-butyle ou un groupe benzyle ; X représente un atome de chlore dans la configuration β (R) ; Y représente un groupe CH₂ ; R₃ et R₄ représentent un groupe OH et n est égal à 1.

7. Composition selon la revendication 6, dans laquelle R₂ représente un groupe méthyle.

8. Composition selon l'une quelconque des revendications 1 à 6, dans laquelle le composé répondant à la formule (I) est présent en une concentration entre environ 0,00002 et environ 0,5 % en poids.

9. Composition selon la revendication 8, dans laquelle le composé répondant à la formule (I) est présent en une concentration entre environ 0,0001 et environ 0,1 % en poids.

10. Composition selon la revendication 9, dans laquelle le composé répondant à la formule (I) est présent en une concentration entre environ 0,001 et environ 0,05 % en poids.

11. Utilisation de la composition selon l'une quelconque des revendications 1 à 6 pour la préparation d'un médicament pour l'administration locale de ladite composition à un oeil glaucomateux ou hypersensible.

12. Utilisation selon la revendication 11, dans laquelle on administre le composé répondant à la formule (I) à concurrence d'environ 0,001 à environ 1000 microgrammes.

13. Utilisation selon la revendication 12, dans laquelle on administre le composé répondant à la formule (I) à concurrence d'environ 0,01 à environ 100 microgrammes.

14. Utilisation selon la revendication 13, dans laquelle on administre le composé répondant à la formule (I) à concurrence d'environ 0,05 à environ 50 microgrammes.
